**Europäisches Patentamt**

**European Patent Office**

**Office européen des brevets**

(19)

(11) Veröffentlichungsnummer : **0 543 424 A2**

## (12) EUROPÄISCHE PATENTANMELDUNG

(21) Anmeldenummer : **92119926.1**

(22) Anmeldetag : **23.11.92**

(51) Int. Cl.$^5$ : **G09B 23/28,** G09B 5/02

(30) Priorität : **22.11.91 DE 4138383**

(43) Veröffentlichungstag der Anmeldung :
**26.05.93 Patentblatt 93/21**

(84) Benannte Vertragsstaaten :
**CH DE ES FR GB IT LI**

(71) Anmelder : **Zirm, Mathias, Dr., Univ. prof.
Fallmerayer Strasse 3
A-6010 Innsbruck (AT)**

(72) Erfinder : **Zirm, Mathias, Dr., Univ. prof.
Fallmerayer Strasse 3
A-6010 Innsbruck (AT)**

(74) Vertreter : **Riebling, Peter, Dr.-Ing.,
Patentanwalt
Rennerle 10, Postfach 31 60
W-8990 Lindau/B. (DE)**

(54) **Verfahren und Vorrichtung zur Durchführung von experimentellen Augenoperationen.**

(57)   Ein Verfahren zur Durchführung von experimentellen Augenoperationen sieht vor, daß das an der Rückseite aufgeschnittene Auge in einer Augenhalterung eingespannt wird und von einer oder mehreren Beleuchtungsquellen angestrahlt wird. Eine oder mehrere Videokameras sind von der Rückseite des aufgeschnittenen Auges her in das Innere des Auges gerichtet, um die Instrumentenbewegungen im Inneren des Auges für einen von der Vorderseite des Auges her operierenden Chirurgen auf dem Videobildschirm sichtbar zu machen.

   Um dem Operateur den Eindruck einer - nicht vorhandenen - Netzhaut zu vermitteln wird von der Unterseite in das Augeninnere ein Rotlicht eingestrahlt, das die entsprechend durchblutete Netzhaut simuliert.

FIG 1

EP 0 543 424 A2

EP 0 543 424 A2

Die Erfindung betrifft ein Verfahren zur Durchführung von experimentellen Augenoperationen nach dem Oberbegriff des Patentanspruches 1, sowie eine Vorrichtung nach dem Oberbegriff des selbständigen Anspruches 5.

Experimentelle Augenoperationen werden entweder an künstlichen Augen oder an Tieraugen durchgeführt, mit dem Zweck, bestimmte Operationstechniken am lebenden Auge zu trainieren.

Ein wichtiges Anwendungsgebiet ist die Star-Operation, bei der es darauf ankommt, im Augeninneren bestimmte Manipulationen auszuführen, die von der Vorderseite (Pupillenseite) des Auges nicht sichtbar sind. Der Operateur muß also teilweise "blind" arbeiten und es hat sich als sehr hilfreich herausgestellt, derartige, schwierige Operations-techniken erst an Trainingsgeräten zu üben, an denen die vorher erwähnten experimentellen Augenoperationen übungsweise durchgeführt werden.

Um den Eingriff der chirurgischen Instrumente in das Augeninnere auch darstellen zu können, ist es bekannt, bei der experimentellen Augenoperation das Auge an der Rückseite aufzuschneiden, um so das Augeninnere von hinten zu öffnen, um sodann in diesen von hinten geöffneten Bulbus eine Video-kamera zu richten, die dem von der Vorderseite her operierenden Chirurgen die Führung seiner Instrumente im Innenraum des Auges zeigt.

Hierbei ist es im übrigen bekannt, das Auge mindestens von unten im Innenraum mit Weißlicht zu bestrahlen und gegebenenfalls auch von oben zu bestahlen, um eine ausreichende Operationshelligkeit zu erhalten.

Eine derartige experimentelle Augenoperationstechnik vermag jedoch bisher nicht genau die natürlichen Verhältnisse bei der Augenoperation nachzubilden. Dadurch, daß der hintere Teil des Bulbus weggeschnitten ist, um den Innenraum vom der Rückseite her für die Videokamera zugänglich zu machen, besteht der Nachteil, daß der größte Teil der Netzhaut fehlt und demzufolge der von der Vorderseite her in das Auge blickende Operateur nicht die gewohnte Netzhautumgebung mit einem entsprechenden Rotlichteffekt sieht.

Ziel der vorliegenden Erfindung ist es, ein Verfahren der eingangs genannten Art so weiterzubilden, daß möglichst naturgetreu die Verhältnisse bei der "wirklichen" Augenoperation nachgebildet werden und daß ferner mit der Videokamera ein großes Untersuchungsfeld im Auge wählbar betrachtet werden kann.

Zur Lösung der gestellten Aufgabe dient die technische Lehre des Anspruches 1.

Wesentliches Merkmal der Erfindung ist, daß nun der gewohnte "Netzhautanblick" durch Erzeugung eines Rotlichtes im Innenraum des Auges nachgebildet wird. Hierzu ist vorgesehen, daß in das Innere des Auges ein Licht mit hohem Rotlichtanteil projiziert wird.

Damit wird die (vorher abgeschnittene und entfernte) Netzhaut durch eine Rotlichtquelle nachgeahmt, was dem Operateur den Eindruck vermittelt, eine Operation am "wirklichen" Auge durchzuführen.

In einer bevorzugten Vorrichtung zur Ausübung des Verfahrens ist vorgesehen, daß im Strahlengang der Videokamera ein Filter angeordnet ist, welcher das Licht mit hohem Rotlichtanteil ausfiltert und lediglich Weißlicht passieren läßt. Damit wird der Vorteil erzielt, daß die Kamera naturgetreu, wie bei einer wirklichen Operation, nur den Weißlichtanteil empfängt und das Innere des Auges auf dem Video-Bildschirm naturgetreu abgebildet werden kann, wo hingegen der Operateur, der von der Vorderseite (Pupillenseite) in das Auge blickt, aufgrund des projizierten Rotlichtes vermeint, eine Netzhaut vor sich zu haben.

In einer bevorzugten Ausführungsform der Erfindung ist vorgesehen, daß eine Rotlichtquelle das Licht auf einen halbdurchlässigen Spiegel stahlt, der zwar im direkten Strahlengang für das Rotlicht durchgängig ist, wobei in Verlängerung dieses Strahlenganges der Rotlichtquelle das zu untersuchende Auge angeordnet ist, wo hingegen die Videokamera mit ihrem Strahlengang das von diesem Spiegel reflektierte Licht empfängt.

Damit wird mit dem halbdurchlässigen Spiegel, der zwar für Rotlicht direkt lichtdurchlässig ist, nicht aber für Weißlicht, die gewünschte Farbmischung mit Rotlicht und Weißlicht zur Simulation einer Netzhaut vorgesehen, wo hingegen die Videokamera lediglich Weißlicht empfängt.

Unabhängig von diesem Verfahren und der Vorrichtung zur Erzeugung eines Rotlichtes im Inneren des Auges wird für die nachfolgende Vorrichtung selbständiger Schutz beansprucht. Die nachfolgend beschriebene Vorrichtung arbeitet also auch ohne die Vorrichtung zur Erzeugung eines Rotlichtanteils im Inneren des Auges.

Bei dieser Vorrichtung ist nämlich vorgesehen, daß im Stahlengang zwischen der Videokamera und dem Auge der oben erwähnte reflektierende Spiegel angeordnet ist. Hier muß nun nicht mehr lösungsnotwendig dieser Spiegel halbdurchlässig für einen Rotlichtanteil sein, denn bei dieser nachfolgend beschriebenen Vorrichtung kommt es nur darauf an, daß ein reflektierender Spiegel im Strahlengang zwischen der Videokamera und dem Auge angeordnet ist. Mit der Anordnung eines derartigen Spiegels, besteht nämlich der wesentliche Vorteil, daß er einstellbar und feststellbar ausgebildet sein kann, so daß nun erfindungsgemäß nicht mehr die Kamera geneigt oder verschwenkt werden muß, um verschiedene Areale im Inneren des Auges zu betrachten, sondern es reicht aus, den Spiegel entsprechend zu verschwenken, um unterschiedliche Areale im Inneren des zu untersuchenden Auges zu betrachten.

Vorteil dieser Maßnahme ist, daß nun die Videokamera zusammen mit einem gegebenenfalls dem Kameraobjektiv vorgeschalteten Mikroskop auf einem XY-Schlitten angeordnet ist. Die X-Richtung wäre dann beispielsweise parallel zu dem Strahlengang der Videokamera und die Y-Richtung ist senkrecht hierzu angeordnet. Damit besteht die Möglichkeit, die Kamera durch die Bewegung des Schlittens in X-Richtung in einfacher Weise scharf zu stellen, wo hingegen bei einer Verstellung der Kamera in Y-Richtung unterschiedliche Untersuchungsfelder vom Inneren des Auges in die Videokamera projiziert werden. Die Kamera wird also in X- und Y-Richtung zu dem reflektierenden Spiegel verstellt.

Die Verstellung des Spiegels kann von außen her erfolgen und z.B. über einen Bowdenzug oder dergleichen durchgeführt werden. Hierbei ist vorgesehen, daß auch der Spiegel in X- und Y-Richtung verschenkbar ausgebildet ist und demzufolge in einer kardanischen Aufhängung gelagert ist.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander. Alle in den Unterlagen - einschließlich der Zusammenfassung - offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von lediglich einen Ausführungsweg darstellende Zeichnungen näher erläutert. Hierbei gehen aus den Zeichnungen und ihrer Beschreibung weitere erfindungswesentliche Merkmale und Vorteile der Erfindung hervor.

Es zeigen:

Figur 1:    schematisiert gezeichnete Seitenansicht einer Vorrichtung nach der Erfindung unter Weglassung verschiedener Funktionsteile,

Figur 2:    die Draufsicht auf die Anordnung nach Figur 1,

Figur 3:    Schnitt durch die Anordnung nach Figur 1 in Höhe der Kamerahalterung,

Figur 4:    Schnitt durch den Schlittenantrieb für die Kamerahalterung,

Figur 5:    perspektivische Seitenansicht der Anordnung, ähnlich wie Figur 1, unter Hinzufügung von Fuktionsteilen,

Figur 6:    Draufsicht auf die Anordnung nach Figur 5.

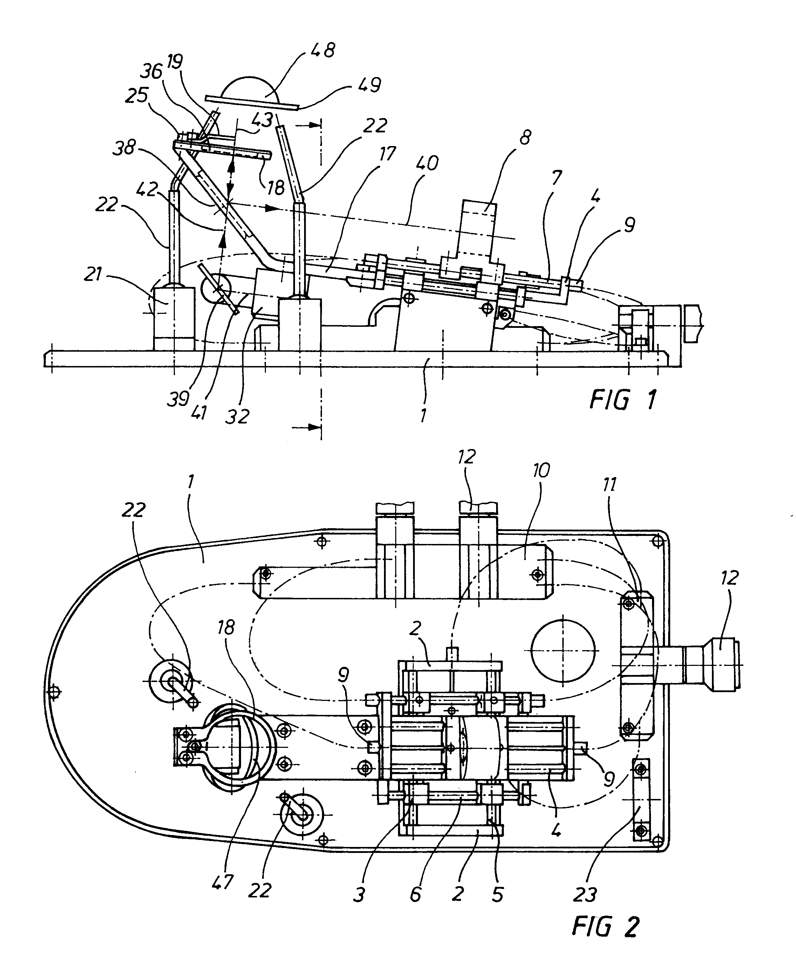

Gemäß den Figuren 1 bis 4 sind auf einer Bodenplatte 1 die Funktionsteile der erfindungsgemäßen Vorrichtung angeordnet. In den Figuren 1 bis 4 ist die Videokamera 45, die Beleuchtungseinrichtung und andere Funktionsteile nicht dargestellt. Die Kamera wird hierbei in einer Kamerahalterung 8 aufgenommen, die im wesentlichen in der Art einer Schelle ausgebildet ist. Dieser Kamerahalter ist hierbei auf einer X-Y-Schlittenanordnung gelagert, wobei der obere Schlitten parallel zueinander verlaufende Führungsstangen 7 aufweist die eine Längsführung 4 parallel zum Strahlengang 40 der Videokamera 45 bilden.

Der Kamerahalter 8 ist in dem oberen Schlitten (d.h. also im Bereich der Führungsstangen 6) verschiebbar parallel zum Strahlengang 40 der Kamera ausgebildet. Zur Verschiebung des Kamerahalters 8 in dem oberen Schlitten (längs der Führungsstangen 6) sind hierbei Seilführungsbuchsen 9 vorgesehen, an welche entsprechende Bowdenzüge angreifen, die in Verstellgetrieben enden, an denen die zugeordneten Bedienungsknöpfe 12 angeordnet sind.

Unterhalb des oberen Schlittens, der entlang den Führungsstangen 6 verschiebbar ist, ist eine Querführung 2 vorhanden, der Führungsstangen 5 zugeordnet sind. Hierdurch wird also ein zweiter Schlitten gebildet, der senkrecht zu dem Strahlengang 40 der Videokamera 45 verschiebbar ausgebildet ist. Die Verschiebung dieses zweiten Schlittens erfolgt wiederum über die vorher erwähnten Bowdenzüge und die Bedienungsknöpfe 12.

Diese Bedienunsknöpfe 12 wirken auf die Zahnstangengehäuse 10 und 11 ein, an deren Ausgang dann die erwähnten Bowdenzüge ansetzen.

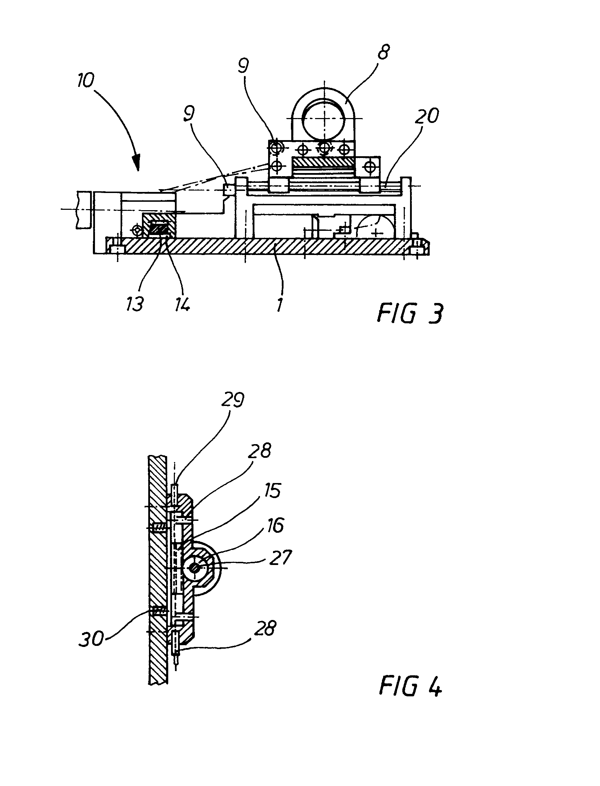

Aus Figur 3 sind noch Einzelheiten eines Zahnstangengehäuses 10 entnehmbar, wo erkennbar ist, daß eine Zahnstange 13 mit einer Gleitplatte 14 zusammenwirkt und daß der Bedienungsknopf 12 drehfest mit einem nicht mehr dargestellten Ritzel verbunden ist, welcher die Zahnstange 13 bewegt.

Eine gleiche Zahnstangenanordnung ist im übrigen für die Verschiebung des Kamerahalters 8 in Richtung des Strahlenganges 40 in Figur 4 dargestellt. Hier ist erkennbar, daß ein Ritzel 16 mit einer in Richtung des Strahlenganges 40 verstellbar angeordneten Zahnstange 15 kämmt und hierdurch der gesamte Kamerahalter verschiebbar ist. Die gesamte Anordnung ist auf der Achse 27 verschiebbar. Seitlich setzen wiederum die vorher erwähnten Seilführungsbuchsen 28,29 an, um die gesamte Anordnung in Verstellrichtung zu verstellen.

Mit der Bodenplatte 1 ist ein Spiegelträger 17 verbunden, der in seinem abgeköpften Bereich einen Spiegel 38 trägt. Es wurde bereits schon vorstehend erwähnt, daß der Spiegel sowohl durchlässig für Rotlicht sein kann als auch nicht, wobei für beide Ausführungsformen gesonderter Schutz beansprucht wird. Der Spiegelträger 17 ist bevorzugt mit dem in X- und Y-Richtung verstellbaren Kamerahalter 8 verstellbar verbunden.

Nachfolgend wird die Rotlicht-Anordnung näher beschrieben.

Unterhalb des Spiegelträgers 17 ist eine Rotlichtquelle 32 angeordnet, die in einer ersten Ausführungsform direkt ihr Licht auf den halbdurchlässigen Spiegel 38 richten könnte.

Im gezeigten Ausführungsbespiel wird jedoch das Licht der Rotlichtquelle 32 (Stahlengang 41) an einem weiteren Spiegel 39 umgelenkt, so daß dessen Strahlengang 42 auf die Rückseite des halbdurchlässigen Spiegels 38 trifft und von diesem unmittelbar durchgelassen wird.

Oberhalb des halbdurchlässigen Spiegels 38 ist am Spiegelträger 17 ein Halter für eine Sammellinse 36 vorgesehen, wöbei diese Sammellinse mit einer Haltefeder 19, die mit Schrauben 25 am Spiegelträger 17 befestigt ist, in Position gehalten wird.

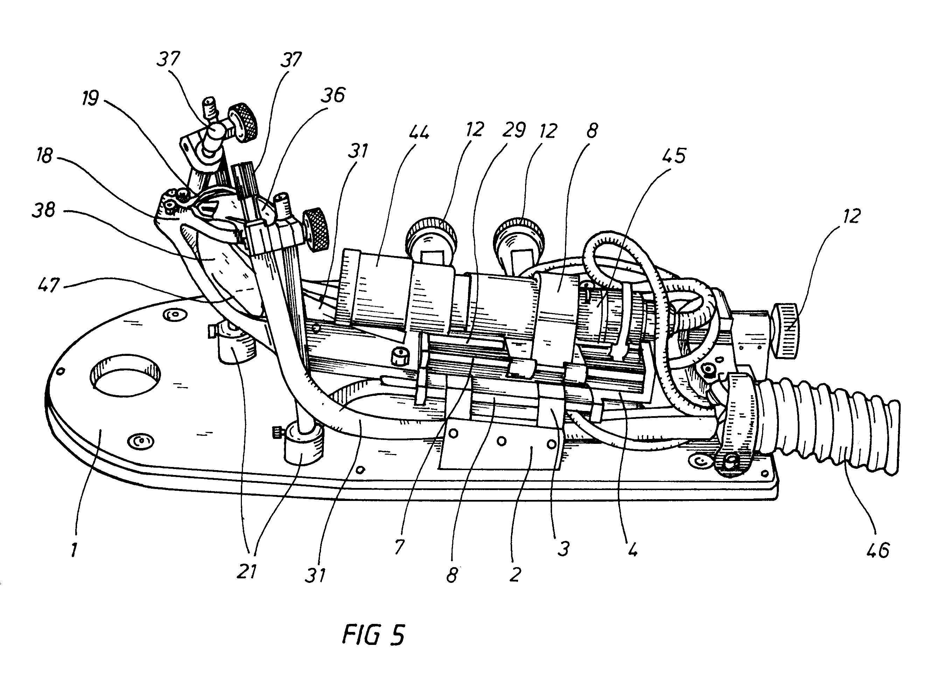

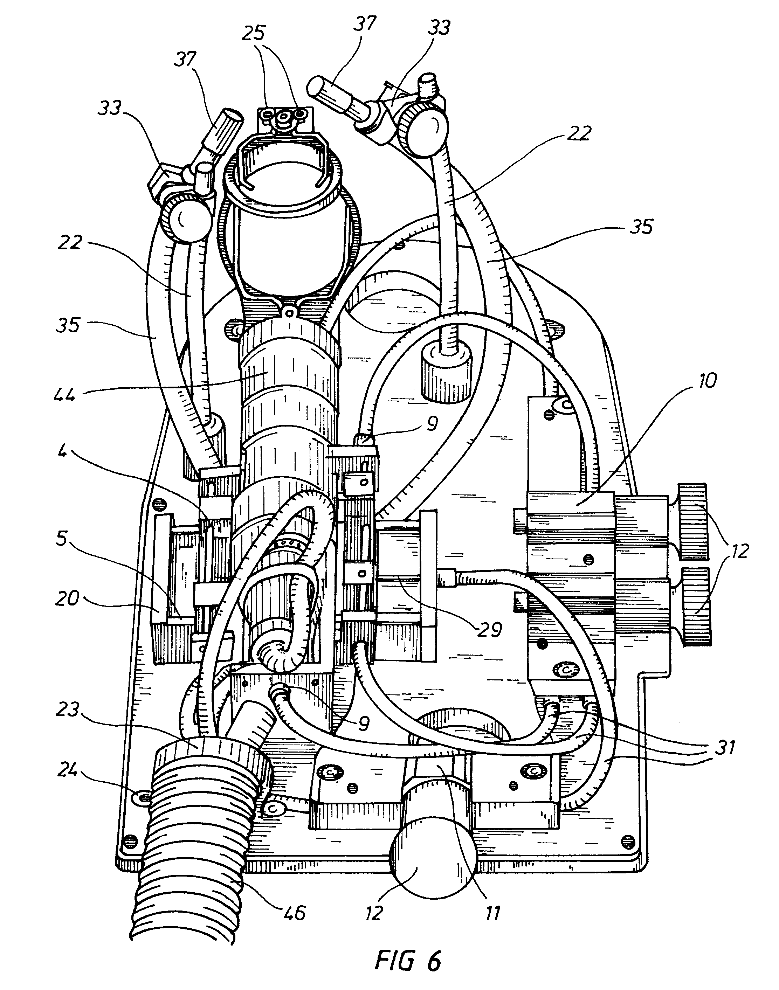

Auf der Bodenplatte 1 sind ferner Sockel 21 zur Aufnahme von abgekröpften Tragarmen 22 vorgesehen, an deren freien Enden mit Hilfe von Schellen (vergleiche Figuren 5 und 6) Schläuche 31 von Faseroptiken befestigt sind. Hierdurch werden gemäß den Figuren 5 und 6 Beleuchtungsköpfe 37 gebildet, die von unten her in das von unten geöffnete Auge 48 einstrahlen, welches auf einer Augenhalterung 49 befestigt ist.

Das Licht für die Faseroptiken (Schläuche 31), für die Rotlichtquelle und die elektrischen Kameraverbindungen werden durch einen Schutzschlauch 46 nach außen geführt, wo die entsprechenden Beleuchtungseinrichtungen vorgesehen sind. Der Schutzschlauch 46 wird hierbei mit einer Schelle 23 am Grund an der Bodenplatte 1 befestigt. Der halbdurchlässige Spiegel 38 wird im Spiegelträger 17 mit Hilfe einer Haltefeder 47 (Figur 5) gehalten.

Vor dem Objektiv der Videokamera 45 ist ein Mikroskop 44 befestigt. Wichtig ist nun, daß im Strahlengang 43 unterhalb des geöffneten Auges 48 Rotlicht und Weißlicht in das Innere des Auges projiziert wird, so daß dem von oben operierenden Chirurgen der Eindruck einer vorhandenen Netzhaut vermittelt wird.

Im Strahlengang 40 der Videokamera ist jedoch eine Rotlichtfilterfolie angeordnet, die das ansonsten in die Videokamera eindringende Rotlicht herausfiltert, so daß diese Videokamera nur Weißlicht empfängt.

Wichtig ist nun, daß - im Ausführungsbeispiel nicht näher dargestellt - der halbdurchlässige Spiegel 38 einstellbar und feststellbar ausgebildet sein kann, um zu gewährleisten, daß durch die entsprechende Ausrichtung des Spiegels unterschiedliche Areale im Innenraum des zu untersuchenden Auges auf die Videokamera abgebildet werden können. Dies ist ein wichtiges Merkmal, welches unabhängig von dem vorher beschriebenen Verfahren und der Vorrichtung zur Erzeugung eines Rotlichtanteils im Inneren des Auges funktioniert.

Für beide Verfahren und Vorrichtungen wird demzufolge gesonderter Schutz in Alleinstellung beansprucht.

**ZEICHNUNGSLEGENDE**

| | | | |
|---|---|---|---|
| 1 | Bodenplatte | 36 | Sammellinse |
| 2 | Querführung | 37 | Beleuchtungskopf |
| 3 | Mittelteil | 38 | halbdurchlässiger Spiegel |
| 4 | Längsführung | 39 | Spiegel |
| 5 | Führungsstange | 40 | Strahlengang |
| 6 | Führungsstange | 41 | Strahlengang |
| 7 | Führungsstange | 42 | Strahlengang |
| 8 | Kamerahalter, 8a Klemmring | 43 | Strahlengang |
| 9 | Seilführungsbuchse | 44 | Mikroskop |
| 10 | Zahnstangengehäuse | 45 | Videokamera |
| 11 | Zahnstangengehäuse | 46 | Schutzschlauch |
| 12 | Bedienungsknopf | 47 | Haltefeder |
| 13 | Zahnstange | 48 | Auge |
| 14 | Gleitplatte | 49 | Augenhalterung |
| 15 | Zahnstange | | |
| 16 | Ritzel | | |
| 17 | Spiegelträger | | |
| 18 | Linsenträger | | |
| 19 | Haltefeder | | |
| 21 | Sockel | | |
| 22 | Tragarm | | |
| 23 | Schelle | | |
| 25 | Schraube | | |
| 27 | Achse | | |
| 28 | Seilführungsbuchse | | |
| 29 | Seilführungsbuchse | | |
| 30 | Feder | | |
| 31 | Schlauch | | |
| 32 | Rotlichtquelle | | |

**Patentansprüche**

1. Verfahren zur Durchführung von experimentellen Augenoperationen bei dem das an der Rückseite aufgeschnittene Auge in einer Augenhalterung eingespannt ist und von einer oder mehreren Beleuchtungsquellen angestrahlt ist, wobei eine oder mehrere Videokameras von der Rückseite des aufgeschnittenen Auges her mindestens ein Bild des Augeninnenraumes erzeugen, **dadurch gekennzeichnet,** daß in das Innere des Auges (48) ein Licht mit hohem Rotlichtanteil projiziert wird.

2. Verfahren nach Anspruch 1, **dadurch gekennzeichnet,** daß im Strahlengang (40) der Videokamera (45) ein Filter (Spiegel 39) angeordnet ist, welcher das Licht mit hohem Rotlichtanteil passieren läßt und lediglich Weißlicht in die Videokamera (45) reflektiert.

3. Vorrichtung zur Ausübung des Verfahrens nach Anspruch 1 oder 2, **dadurch gekennzeichnet,** daß eine Rotlichtquelle (32) das Licht auf einen halbdurchlässigen ersten Spiegel (38) strahlt, der für das Rotlicht durchgängig ist, daß in Verlängerung des Strahlengangs (42) der Rotlichtquelle (32) das zu untersuchende Auge (48) angeordnet ist und daß die Videokamera (45) mit ihrem Strahlengang (40) das von dem ersten Spiegel (38) reflektierte Licht empfängt.

4. Vorrichtung nach Anspruch 3, **dadurch gekennzeichnet,** daß die optische Achse der Rotlichtquelle (32) etwa parallel zum Strahlengang (40) der Videokamera angeordnet ist und daß der Strahlengang (41) der Rotlichtquelle (32) an einem zweiten verstellbaren Spiegel (39) umgelenkt wird.

5. Vorrichtung zur Durchführung von experimentellen Augenoperationen bei dem das an der Rückseite aufgeschnittene Auge in einer Augenhalterung eingespannt ist und von einer oder mehreren Beleuchtungsquellen angestrahlt ist, wobei eine oder mehrere Videokameras von der Rückseite des aufgeschnittenen Auges her mindestens ein Bild des Augeninnenraunes erzeugen, **dadurch gekennzeichnet,** daß im Strahlengang (40,43) zwischen der Videokamera (45) und dem Auge (48) ein reflektierender Spiegel (38) angeordnet ist.

6. Vorrichtung nach Anspruch 5, **dadurch gekennzeichnet,** daß der Spiegel (38) verstellbar und feststellbar ist.

7. Vorrichtung nach einem der Ansprüche 3 bis 6, **dadurch gekennzeichnet,** daß vor der Videokamera (45) ein Mikroskop (44) angeordnet ist.

8. Vorrichtung nach einem der Ansprüche 3 bis 7, **dadurch gennzeichnet,** daß die Videokamera (45) in zwei zueinander senkrechten Achsen einstellbar und verschiebbar auf einer XY-Schlittenanordnung angeordnet ist.

9. Vorrichtung nach einem der Ansprüche 3 bis 8, **dadurch gekennzeichnet,** daß als Beleuchtungsquelle für die Bestrahlung des Auges (48) mit Weißlicht mindestens zwei Faseroptiken vorgesehen sind.

10. Vorrichtung nach einem oder mehreren der Ansprüche 3 bis 9, **dadurch gekennzeichnet,** daß ein oder mehrere zusätzliche Sperrfilter vor der Optik des Mikroskops angeordnet sind.

11. Vorrichtung nach einem der Ansprüche 3 bis 10, **dadurch gekennzeichnet,** daß der den halbdurchlässigen Spiegel (38) tragende Spiegelträger (17) verstellbar an dem XY-Schlitten befestigt ist.

FIG 1

FIG 2

FIG 3

FIG 4

FIG 5

FIG 6